# EUROPEAN PATENT SPECIFICATION

(11) **EP 0 578 631 B1**
(45) Date of publication and mention of the grant of the patent: **03.07.1996**
(21) Application number: 90914869.4
(22) Date of filing: 18.09.1990
(51) Int. Cl.: C12Q 1/24

(54) **CELL SEPARATION INVENTION**
TRENNUNG VON VERSCHIEDENEN ZELLEN
SEPARATION DE CELLULES DIFFERENTES

(30) Priority: 20.09.1989 US 410091
(43) Date of publication of application: 19.01.1994
(73) Proprietor: VIVORX, INCORPORATED, Santa Monica, CA 90404 (US)
(72) Inventor: SOON-SHIONG, Patric, Santa Ana, CA 92705 (US)
(74) Representative: Fiener, Josef
(86) International application number: US9005289
(87) International publication number: WO9104318

(56) References cited:
- EP-A- 0 190 488
- US-A- 640 785
- Hoppe-Seyler's Zeitschrift fuer Physiologische Chemie, Vol. 358, issued October 1977, "Isolation of Functionally Intact Pancreatic Islets by Centrifugation in Metrizame Gradients", RAYDT; pages 1369-1373, see entire article.
- Diabetes, Vol. 38, Supplement 1, issued 01 January 1989, D'ALESSANDRO et al.,
- "Use of UW Solution in Pancrease Transplantation", pages 7-9, see entire article.
- Diabetes, Vol. 38, Supplement 1, published 01 January 1989, LAKE et al., "Large Scale Purification of Human Islets Utilizing Discontinuous Albumin Gradient on IBM 2991 Cell Separator", pages 143-145, see paragraph bridging pages 143 and 144.

## Description

### Field of the Invention:

This invention relates to the fields of cell biology and medicine, and more particularly, to the field of mammalian cell separation of, particularly for insulin-producing cells.

### Art Background:

Isolation and purification of a specific cell population is an important issue in many areas of cell biology. Several methods of purification of cells have been used over the years, including centrifugal separation based upon size or density, cloning and immunological (antibody) recognition and separation, among others.

Over the years, many different compounds have been used to form density gradients to enable particles to be separated according to their size and/or buoyant density. Some common materials for this purpose include ficoll, percoll, cesium chloride and dextran. While these materials may have been used for the separation of cells, they have certain drawbacks which make them not particularly well-suited for this task. These drawbacks are due in part to sensitivity of the cells to high osmotic or ionic stress, sensitivity to the toxic compounds used, and stability of the separation compounds to autoclaving or other sterilization techniques.

When cells are separated using centrifugation, they may be separated by size or buoyant density, or to a minor extent, by charge or other related external surface characteristics. With respect to separation processes relying upon differences in size, when particles in solution are subjected to a centrifugal field, they move in the direction of the force applied, and in general, larger particles will move faster than smaller particles. Therefore, when cells differ greatly in size, a reasonable purification may be obtained by low speed centrifugation in a suitable medium.

In cell suspensions containing a mixed population of cells, cells of similar sizes may have different buoyant densities, whereas cells of different sizes may have the same densities. Cells having different buoyancy characteristics may be separated on a density gradients, such as continuous gradients or discrete step (discontinuous) gradients. In either case, the principle is that the cells will migrate through the gradient medium until they reach a point where the density of the medium equals the density of the cells, in the case of a continuous gradient, or where the cells are sandwiched at the interface in between a medium having a lower density and a medium having a greater density than the cells, in the case of a discontinuous gradient. This method results in the cells being disposed in discrete bands within the medium.

An example of the need for rapid isolation and purification of cells is the desirability of acquiring large quantities of pure insulin producing cells from a pancreas for purposes of transplanting them into a diabetic patient. The pancreas is the organ responsible for insulin production. Specifically, insulin is produced and regulated by areas of the pancreas known as the Islets of Langerhans, referred to herein as islet cells or islets, which are the endocrine cells of the pancreas. Such cells comprise a small percentage of the pancreas (around 2%). The major cellular component of the pancreas consists of exocrine tissue including acinar and ductal cells, and it has been shown to be a formidable task to purify the islet cells from the acinar cells. Gray and Morris, Transplantation (1987) 43:321.

Islet cell isolation and purification is currently being performed by density gradient separation based upon the principle of density differences between the isolated islets and the acinar cells. Generally the dispersed pancreatic preparation is placed in a discontinuous density gradient solution containing ficoll®, percoll® or dextran, all well-known density gradient materials. There are several disadvantages to prior art methods of islet cell isolation and preparation utilizing such materials. For one, the method is cumbersome, time consuming and labor intensive since several gradients are required to be layered and the islets must be carefully removed from within these multiple gradients. Another disadvantage is that the method yields inconsistent results since the density of the islet cells and acinar cells may change during the process as a result of edema in the cells caused by the materials used in the separation. Another serious disadvantage of the prior art methods is that the cells are subjected for substantial periods of time to the gradient material which may be toxic, and is at least detrimental to the viability of the cells. This is particularly the case because the currently used gradient solutions such as ficoll®, percoll® and dextran are not physiological solutions thus causing both osmotic and ionic stresses on the cells. Ficoll® is known to be toxic to cells as well as mutagenic. Percoll® also causes cellular damage. Dextran in the concentrations used in gradient separation may cause cellular damage as a result of the osmotic stresses applied to the cells. Furthermore, none of the gradient separation materials are capable of preserving cells for any substantial periods of time.

### Summary of the Invention

The present invention comprises a cell separation material useful in the separation and purification of cells, particularly islet cells, and a method of cell separation using the invented material. The material has properties and characteristics of a physiological solution and therefore is not harmful and is even somewhat beneficial to the cells. Moreover, it has cell preservation properties, being derived in part from a tissue preservation solution.

The invented material is capable of separating two mixed types of cells, being acinar and islet cells, without being entirely dependent upon the naturally occurring densities of the respective cells. Moreover, the multiple gradients required in the prior art separation techniques are not required in the present invention in that an initially two phase aqueous separation system is capable of performing the desired separation.

The composition of this invention is made of heat stable material. This facilitates sterilization by allowing autoclaving.

The present invention is a composition comprising two aqueous phases, as well as a continuous gradient which develops between the two phases, of solutions useful in the isolation and purification of two types of cells from a mixture. In particular, this invention is useful in separating islet cells from a pancreatic tissue digestate containing acinar cells in addition to the islets. The composition permits high viability of at least one type of cells, such as the islet cells, especially under cold conditions (such as 4°C), and permits these cells to be fully biologically active after the separation. The present invention also comprises a method of isolating and purifying islet cells using the composition.

### Brief Description of the Drawings

FIGURE 1 is a schematic view of the method of the present invention.

FIGURE 2 is a graph demonstrating the viability and effectiveness of islet cells obtained by the invented method, which cells were obtained from canine pancreatic tissue and implanted into diabetic rats.

FIGURE 3 is a graph demonstrating the viability and effectiveness of islet cells obtained by the invented method, which cells were obtained from rat pancreatic tissue, encapsulated in an alginate capsule and implanted into diabetic rats. This graph shows that the islet cells implanted in the rat kidneys were functional for at least ten weeks.

FIGURE 4 is a bar graph demonstrating in vitro that the islet cells recovered from the experiment discussed above in connection with Figure 3 after 75 days of implantation were still reactive to glucose by producing insulin.

FIGURE 5 is a graph also demonstrating the effectiveness of the encapsulated islet cells of the experiments of Figures 3 and 4. in vitro by constant perfusion of the encapsulated cells with first 60 mg% and then 300 mg% glucose and then 60 mg% glucose again and the insulin release is measured.

FIGURE 6 is a photograph of a 50 ml centrifuge tube containing rat pancreatic tissue and showing the separation of the acinar cells which are pelleted at the bottom of the tube and the islet cells which banded at the interface near the center.

FIGURE 7 is a 100x microscope photograph of a sample taken from the center (interface) of the tube of FIGURE 6 showing only islet cells.

FIGURE 8 is a 100x microscope photograph of a sample taken from the bottom of the tube of FIGURE 6 showing only acinar cells.

### Detailed Description of the Invention

Both initial phases of the composition can be made from the same solution, but with different densities. This solution is a combination of a density gradient component and an organ preservation solution comprising an osmolarity adjusting agent and a viscosity modifier.

The density gradient component is a water soluble, metabolically inert substance which maintains density, osmolarity and pH in the presence of cells, and is useful in gradient separation. An example is 5-(N-2,3-dihydroxypropylacetamido) 2,4,5-tri-iodo-N,N'-bis(2,3-dihydroxypropyl)isophthalamide sold under the trademark Nycodenz® by Nycomed AS of Norway and Robbins Scientific (Mountain View, California). This material has a molecular weight of 821 and a density of 2.1 g/ml. It is very soluble in water and its osmolarity is near physiological. Unlike ficoll® and percoll®, 5-(N-2,3-dihydroxypropylacetamido) 2,4,5-tri-iodo-N,N'-bis(2,3 dihydroxypropyl)isophthalamide is non-toxic to cells and is heat stable, even in solution, making it autoclavable for purposes of sterilization. Another water soluble, metabolically inert substance which may be used in the present invention is a tri-iodinated benzamide derivative of glucose sold under the trademark Metrizamide, also available from Nycomed AS of Norway and Robbins Scientific (Mountain View, California), which is the International Non-Proprietory Name (INN) for 2-[3-acetamido-2,4,6-triiodo-5-(N-methylacetamido)-benzamido]-2-desoxy-D-glucose.

The 5-(N-2,3-dihydroxypropylacetamido) 2,4,5-triiodo-N,N'-bis(2,3-dihydroxypropyl)isophthalamide or other water soluble, metabolically inert material is dissolved in solution. The aqueous solution in which the water soluble, metabolically inert material is dissolved has a viscosity modifier comprising one or more colloids or polymers so that the solution has a viscosity preferably in the range of approximately 1.5 to 3.2 mPas. Such viscosity modifiers include colloids such as hydroxyethyl starch or serum albumin, or polymers such as polyethylene glycol, dextran and alginate. Alginate can be used in concentrations ranging from 0 to 2.5% of the solution to provide the required viscosity of the solution.

The solutions can be isotonic to the cells to be separated. However, in one aspect of the invention, the second of the two types of cells in the mixture to be separated is more permeable than the first. In this case, if the solutions are somewhat hypertonic, this more permeable second cell type will increase in density with incubation in the hypertonic solution. This aids separation of the two cell types by increasing the difference in density of the two cell types. For example, when the first cell type is islet cells and the second cell type is acinar cells, the acinar cells can preferentially be made more permeable by directing collagenase enzymes to acinar cells via ductal injection of the enzymes. The acinar cells then increase in density with incubation, aiding in cell separation. The islet cells, on the other hand, are preserved in their natural state and are kept viable by this procedure. To effect separation of the cells after incubation, the density of the solution for use in the lower phase of the gradient is made to be heavier than the density of the second type of cells in their natural state, but lighter than the density of the second cell type after they incubate in this solution.

When the solution is made in the range of approximately 290 - 320 mOsm, which is at or very close to the physiological range of osmolarity for most cells, the yield of the desired, less permeable cells is optimized. At an osmolarity of somewhat higher levels, i.e preferably between approximately 320 and 550 mOsm, purity of the desired less permeable cells is optimized. Therefore, in order to achieve a reasonable level of both purity and yield, a range of approximately 320 to 440 mOsm is preferred.

Osmotically active impermeants are osmolarity enhancing compounds which do not permeate into the cells. Such materials are useful in providing a physiological environment for cells without causing cell edema to the preferred cell type, whereas, traditional gradient separation materials contribute to cell edema in all cells exposed to those materials. Osmotically active agents which are beneficial in the invented solution include sugars such as glucose, mannitol, sucrose, anions such as phosphate, sulfate, glycerophosphate, citrate and gluconate, and compounds which specifically prevent or suppress cold-induced edema such as lactobionate acid or raffinose. Organ preservation solutions which could be used are Eurocollins and UW solutions. UW solution, in particular, has been reported as an organ preservation solution for liver and pancreas transplantation (Lancet 7:617-619, 1988) obtained from DuPont Critical Care (Waukegan, Illinois). UW solution is comprised of the following components:

| Ingredient | Concentration/liter |
|---|---|
| K lactobionate | 100 mmol |
| NaKH₂PO₄ | 25 mmol |
| Adenosine | 5 mmol |
| MgSO₄ | 5 mmol |
| Glutathione | 3 mmol |
| Raffinose | 30 mmol |
| Allopurinol | 1 mmol |
| Insulin | 100 U |
| Trimethoprim/sulphamethozazole | 8 mg/40 mg |
| Modified hydroxyethyl starch | 50 g |
| Na 30 mmol/l | |
| K 120 mmol/l | |
| pH 7.4 | |
| 320-330 mmol/l | |

The first, or lower, solution has a density somewhat greater than the density of both cell types in their natural state, in the range of 1.097 to 1.126 g/ml for islet-acinar cell separation. This density will prevent the preferred cells, such as the islets, from entering the lower phase of the gradient. The cells of the second type, which are somewhat permeable to the solute in which they are incubating, as well as cells which are no longer viable due to the preferential degradation through the use of enzymes such as collagenase, become more dense than in their natural state through uptake of the solute. These cells will then enter the lower phase. The lower phase should be at a density at or slightly lighter than the heavier cells become after incubation, so that the heavier cells will in fact fall through the lower phase and settle at or near the bottom of the container. One means to determine the appropriate density for the lower layer is to incubate a crude prep of the heavier cells in various densities of the solution that forms the lower layer. Observing these incubating cells after a period of time long enough for the cells to change in density will show which density is appropriate for the lower phase of the gradient.

The upper phase of the two phase aqueous solution can be made of the same solution as the lower phase, but at a different density. Alternatively, the upper phase can be a physiological solution containing nutrients, such as a culture medium like RPMI, Hank's solution, MEM, Eagles medium, or the like. When the upper phase is made of a different density version of the same solution as the lower phase, a small, third layer of physiological solution is preferably added as an uppermost layer to trap any fat cells contained in the mixed population, such as from tissue digestate.

The density of the upper phase is less than that of both cell types, i.e. for islet-acinar separation is less than 1.050, preferable less than 1.008, and most preferably around 1.004 g/ml. The greater the difference between the densities of the initial two phases of the gradient, the wider the continuous gradient is that forms during separation. This wider continuous gradient region will allow better separation of the two cell types.

As noted above, the subject invention also comprises a method of isolating and purifying one population of cells, such as islet cells, using the invented two phase aqueous solution. The invented method comprises generally the steps of (1) obtaining a mixture of cells, such as a pancreatic digestate, preferably in a manner which preferentially slightly damages one population of cells, such as acinar cells making them more permeable, (2) combining the mixture of cells in the bottom (first) solution in a container such as a centrifuge tube, (3) adding the second solution to the container, with an optional third uppermost layer, as described previously, and (4) allowing separation, either by standing or centrifuging at a low speed for a sufficient time and speed to obtain separation of the two cell types, such as acinar from islet cells, with the denser, or second, cell type, such as damaged or non-viable acinar cells, migrating to or near the bottom of the tube and the lighter, or first, cell type, such as islet cells, collecting at or near the interface between the two solutions. During this separation a short, continuous gradient forms between the two discreet phases, providing a range of densities intermediate between those of the two original phases. The lighter or first type of cells, such as the islet cells, collect at the density within this continuous gradient corresponding to their internal density. Finally, (5) cells of the first, or desired type, such as islet cells, are removed from the gradient where they have formed a band. With an islet-acinar mix, the purity of the islet cells is very high, and may be in the range of 85% to 95%, depending on the preparation and technique.

For islet-acinar separation, the pancreatic tissue may be obtained from any pancreas containing animal, such as pig, cow, dog, rat, monkey or human. The pancreatic tissue is obtained from the donor animal and then digested into a cell suspension by any of various methods known in the art such as the method described in Warnock, et al, Transplantation, 45:957-963, No. 5, May, 1988. These procedures utilize ductal injection of collagenase to preferentially damage acinar cells, which are present surrounding the ducts. The pancreatic tissue is preferably maintained at 4°C or similar cold temperature during the entire isolation and purification process to ensure that the enzymes contained in the acinar cells do not have an opportunity to lyse the islet cells.

The mixed population of cells, such as pancreatic tissue digestate so obtained, is suspended in the first solution which is preferably 5-(N-2,3-dihydroxypropylacetamido) 2,4,5-tri-iodo-N,N'-bis(2,3-dihydroxypropyl)-isophthalamide in UW solution. The mixed population such as the pancreatic tissue is first suspended in about 1 to 5 ml of physiological Hank's or similar aqueous material and then mixed into a volume of the first solution to create the appropriate density. Any container appropriate for the separation of gradient layers can be used. If one rat pancreas is used, about 15 ml of the first solution can be used in a 50 ml centrifuge tube, and if one canine pancreas is used about 150 ml of the first solution can be used in a 250 ml tube.

The second solution, preferably RPMI, is then added to the container. At least approximately 1/4 of the volume of the first solution is layered over the first solution. The gradient can then be allowed to incubate at lg for a period of at least 30 minutes, or until the heavier cells, e.g. the acinar cells, separate and fall to or near the bottom of the container. Alternatively, the container can be centrifuged, preferably for 15 minutes at 4°C at a speed sufficient to create a centrifugal force that does not damage the first; or desired cell type, preferably at a speed sufficient to create a centrifugal force at the interface between the first solution and second solution up to around 180 g. The lighter cells, e.g. the islet cells, are then removed from at or near the interface. The lighter cells thus obtained are a relatively pure culture, being free from the heavier cells, and being relatively viable and healthy.

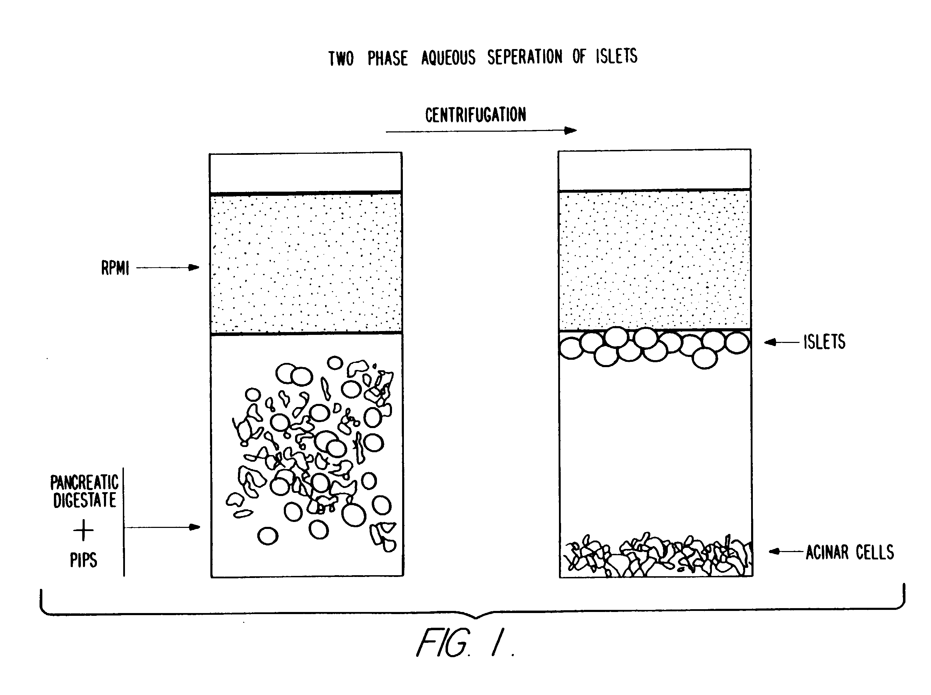

Figure 1 shows a general schematic step diagram of the invented method. The term PIPS stands for the physiological islet purification solution, or the first solution of the present invention. In the pancreatic digestate, the circles represent the islet cells and the smaller irregularly shaped objects are the acinar cells in the mixed population of the digestate.

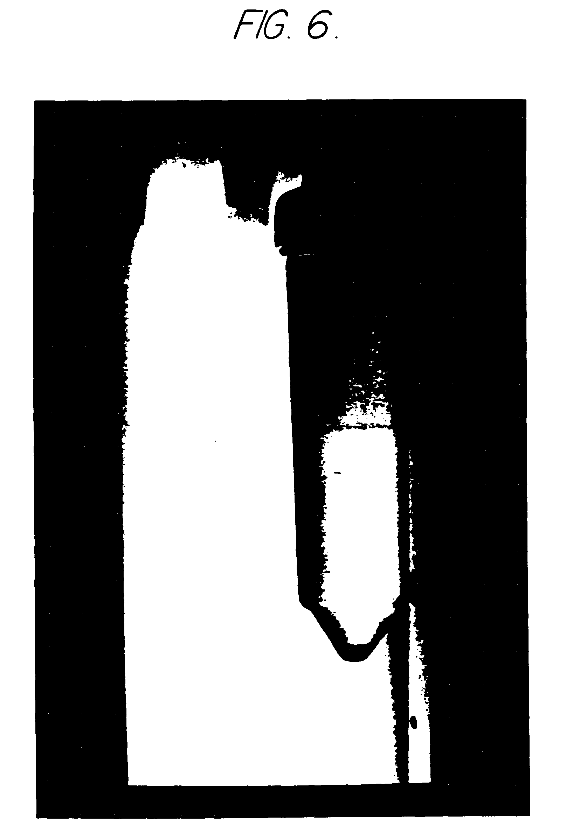

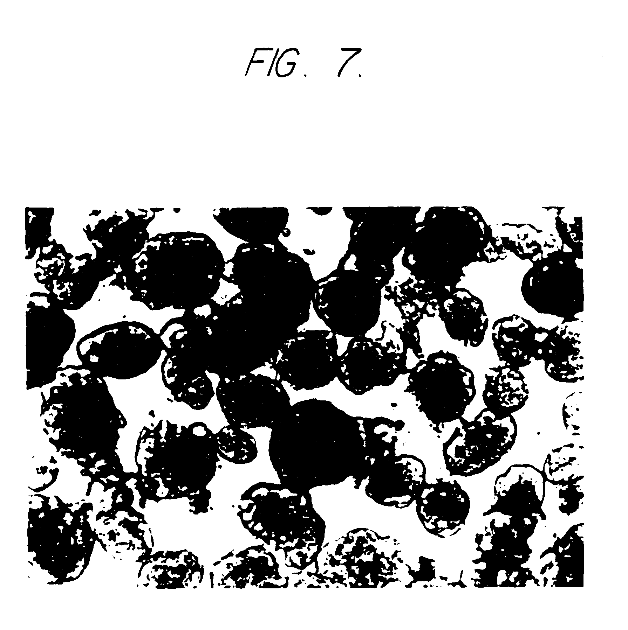

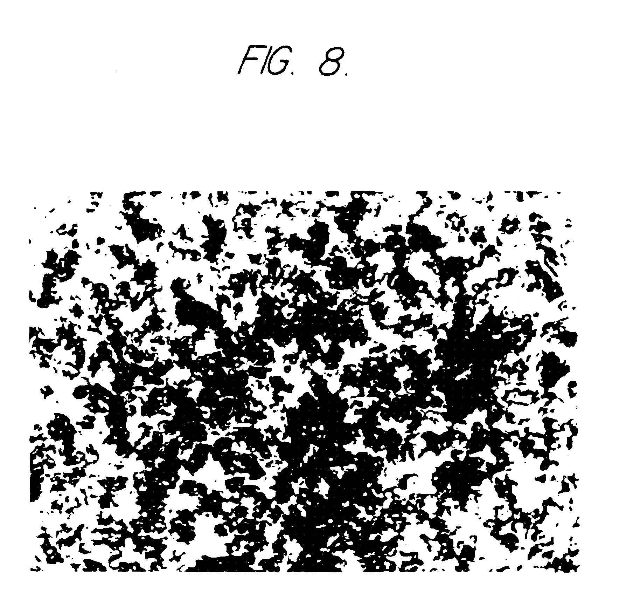

Figures 6, 7 and 8 are several photographs illustrating the method of the present invention. Figure 6 is a photograph of a 50 ml tube after centrifugation containing the two phase aqueous solutions with the islet cells disposed at the interface and the acinar cells disposed in the bottom of the tube. Figure 7 is a photograph of a microscopic view of the cells taken from at or near the interface showing that only the islet cells are present at the interface. Figure 8 is a photograph of a microscopic view of the cells taken from the bottom of the tube showing that only acinar cells are present there.

Several experiments were performed to demonstrate the effectiveness of the subject invention.

### Experiment 1

### Comparative In Vitro Viability

Islet cells were isolated, hand-picked and separated into three groups. Equal numbers of cells were incubated for 30 minutes at 4°C into three different solutions, RPMI, PIPS and ficoll®. The PIPS and ficoll® were both dissolved into UW solution. Both populations were removed from their respective incubation solutions and placed in a physiological solution to measure the cell function in terms of insulin production. The cells were incubated in specified glucose solutions of 60 mg%, which is physiological base line, and 300 mg% which is elevated, and which should induce full cellular production of insulin. The cells were then returned to the 60 mg physiological basal level of glucose. As can be seen from the following Table 1, the cells incubated in PIPS produced over twice the insulin (10.4 compared with 4.6) as the ficoll incubated cells. The numbers in parenthesis are the stimulation index, that is, the ratios between the insulin production compared with the baseline value at the first 60 mg% incubation.

**Table 1**

| INSULIN in µUnits/ml (ratio to baseline) | | | |
|---|---|---|---|
| | Glucose | Glucose | Glucose |
| Incubation Sln. | 60 mg% | 300 mg% | 60 mg% |
| RPMI | 9.7 (1.0) | 15.0 (1.8) | 14.2 (1.5) |
| PIPS | 6.8 (1.0) | 70.8 (10.4) | 26.6 (3.9) |
| Ficoll® | 7.2 (1.0) | 32.9 (4.6) | 23.9 (3.3) |

### Experiment 2

### Transplantation of Purified Human Islets

Human islets were isolated in accordance with this invention. A nude rat was transplanted with 4000 human islets in a blood clot under the renal capsule. Four weeks post-transplant, the rat was anesthetized and cannulated for systematic blood sampling. Heparinized saline (10 units/ml) was used in the cannula to replace blood volume and 0.3 to 0.4 ml of blood was removed at time 0, 5, 15, 30, 45 and 60 minutes after glucose injection of 3 mg/ml of body weight. Plasma was frozen for subsequent RIA of insulin and blood glucose measurements in accordance with procedures known in the art. Blood was analyzed in duplicate. The results are set forth in Table 2 below. The table shows the insulin and blood glucose concentrations from the experiment at the specified time intervals. As can be seen, high levels of insulin release occur in response to glucose stimulation, demonstrating excellent viability of islets isolated in the invented solution utilizing the invented method.

**Table 2**

| Time (min) | Insulin (ng/ml) | Blood Glucose (mg/ml) |
|---|---|---|
| 0 | 0.774 | 212 |
| 5 | 1.120 | 336 |
| 15 | 1.167 | 408 |
| 30 | 2.585 | 498 |
| 45 | 2.911 | 467 |
| 60 | 3.585 | 590 |

### Experiment 3

### Transplantation of Purified Dog Islets

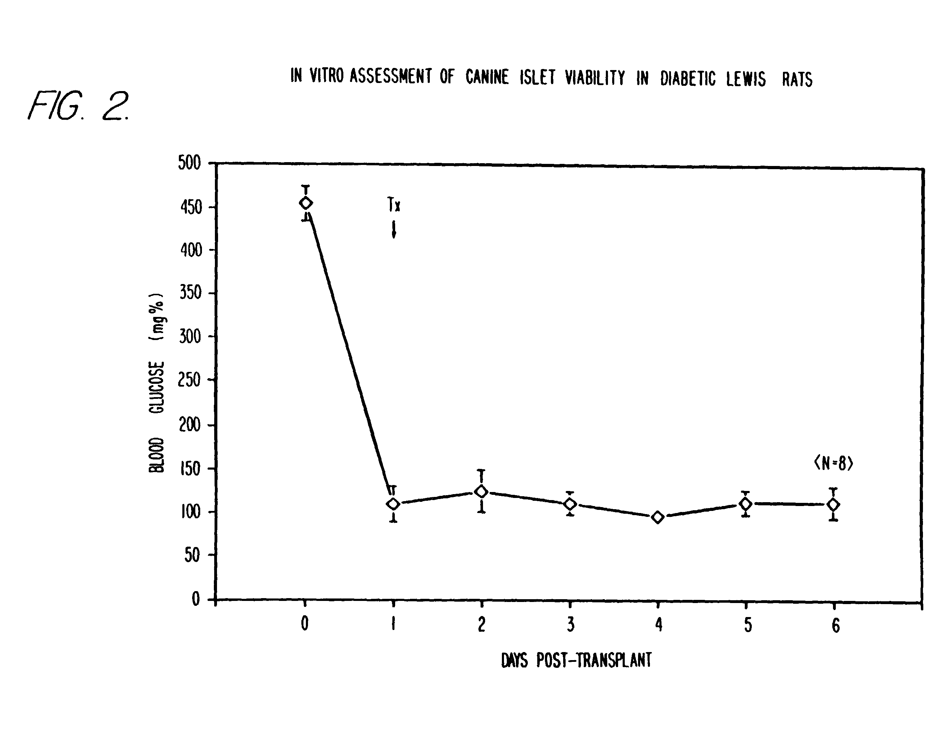

Islet cells obtained from canine pancreas in accordance with the present invention were encapsulated in accordance with procedures described by Lim and Sun in order to prevent immunological rejection due to histocompatibility differences resulting from the use of the cells in a different species. The encapsulated islets were then transplanted into a diabetic Lewis rat. The blood glucose levels were then monitored on a daily basis. As shown by Figure 2, the initial blood glucose level was greater than 450 mg%. After transplantation of the encapsulated islets, the level dropped to approximately 100 mg% and remained at that level for the 6 days for the experiment, demonstrating in vivo viability of the canine islets isolated using the present invention.

### Experiment 4

### Longterm In Vivo Viability

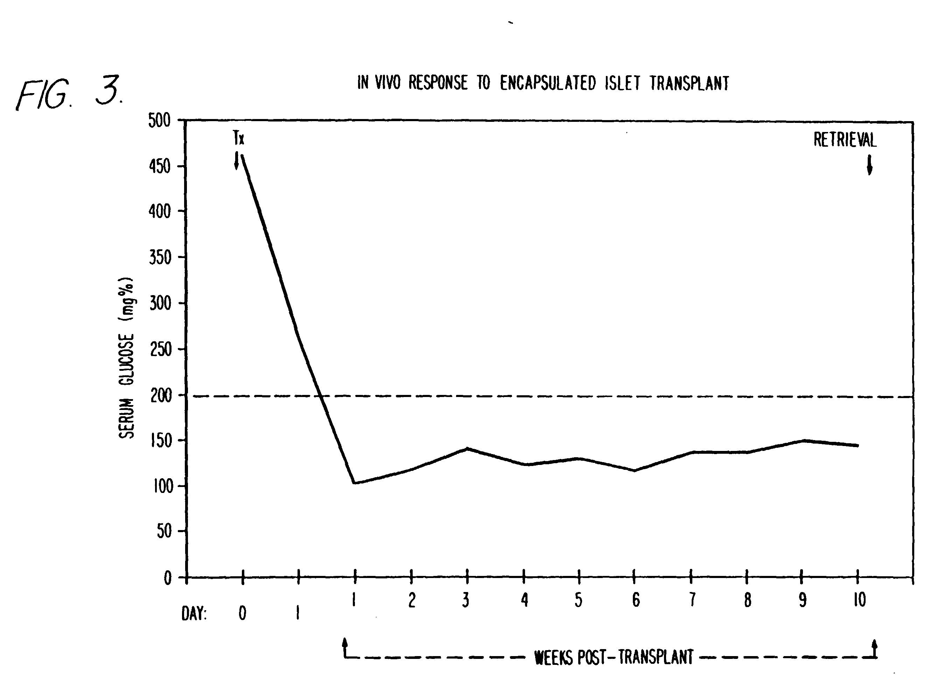

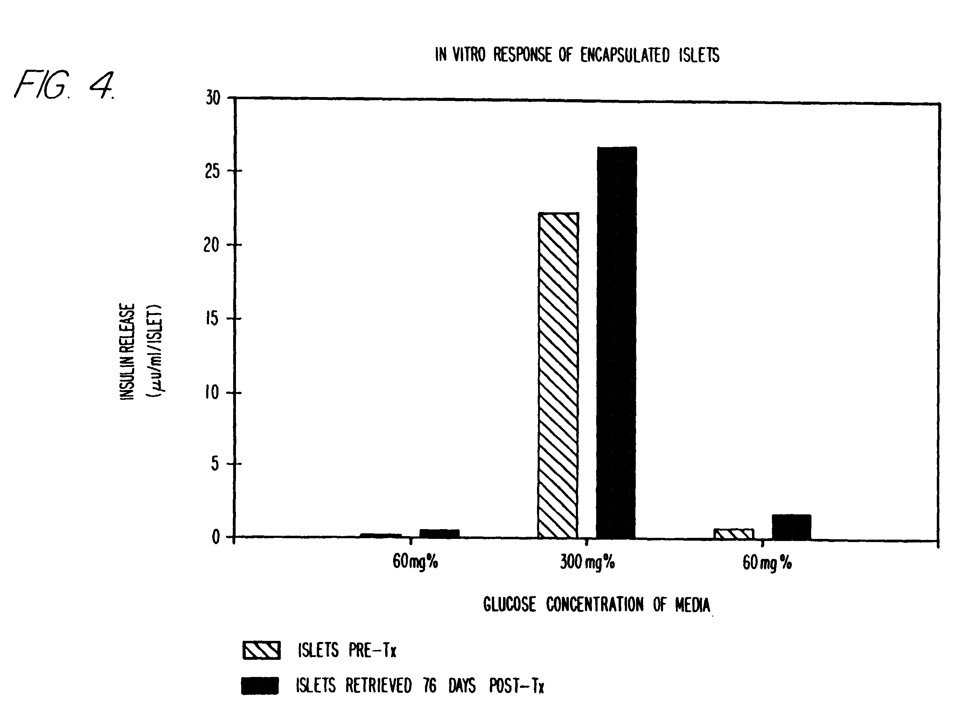

Figures 3, 4, and 5 show the result of an experiment wherein rat islet cells purified in accordance with the present invention. The islet cells were encapsulated as discussed above and implanted into diabetic Lewis rats for up to 75 days. Figure 3 shows that the islet cells functioned for the entire length of the experiment in that the serum glucose levels dropped from over 450 mg% prior to implantation to 100 - 150 mg% and remained at that level for the 75 days until the experiment was terminated.

The encapsulated cells were then removed from the diabetic rat into which they were transplanted and the function of the islet cells was then tested in vitro. The cells were exposed to 60 mg% glucose, 300 mg% glucose and then 60 mg% glucose and the production of insulin was measured. These results were compared with islets that were tested prior to transplantation. As can be seen from the bar graph, the 300 mg% incubation caused the islets to release substantial amounts of insulin and the post transplanted and pre-transplanted cells functioned nearly the same, and in fact the post transplantation cells functioned slightly better.

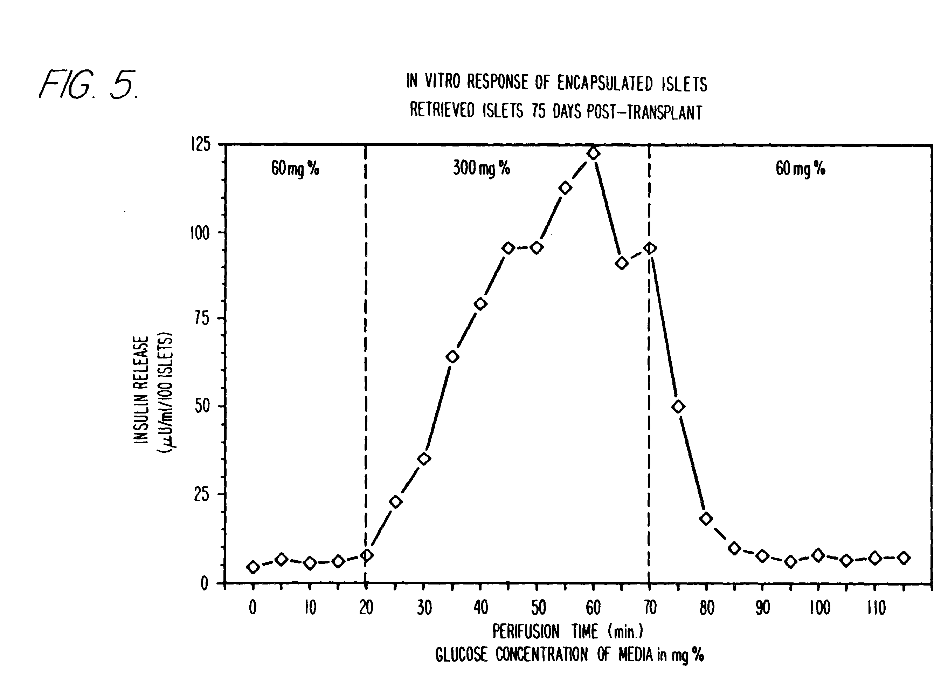

In a similar experiment, the results of which are shown in Figure 5, the post-transplanted islets were perfused with a solution containing varying concentrations of glucose. The cells effectively produced insulin after exposure to 300 mg% glucose and the production of insulin decreased immediately after the glucose concentration in the perfusion medium was decreased to 60 mg% again.

## Claims

1. An aqueous composition, initially present as a two-phase gradient, useful in the isolation and purification of two cell types having different densities, wherein a first cell type, being acinar cells, has a higher density than a second cell type, being islet cells, after incubation in the first or lower solution, said composition comprising:
A) a first, or lower solution having a density no higher than the density of the first cell type, optionally between 1.097 and 1.126 g/ml, said first solution comprising
1) a water soluble, metabolically inert substance which maintains density, osmolarity and pH in the presence of cells, optionally selected from the group 5-(N-2,3-dihydroxypropylacetamido) 2,4,5-tri-iodo-N,N'-bis(2,3-dihydroxypropyl)isophthalamide and Metrizamide ;
2) mixed with a physiological cold storage solution, optionally selected from UW solution and Eurocollins, comprising
a) viscosity modifiers, optionally selected from colloids, optionally selected from the group hydroxyethyl starch and serum proteins; and polymers, optionally selected from the group polyethylene glycol, dextran and alginate; to increase the viscosity of said first solution, and b) osmotically active impermeant agents for suppressing cold-induced cellular edema, optionally selected from sugars, optionally selected from the group glucose, mannitol, and sucrose; anions, optionally selected from the group phosphate, sulfate, glycerophosphate, citrate and gluconate; and compounds which supress cold induced cellular edema, optionally selected from lactobionate acid and raffinose;
B) and a second, or upper solution having a density less than the second cell type, optionally less than 1.050 g/ml, said second solution selected from the group comprising:
1) a physiological solution capable of maintaining viable cells, optionally selected from Hank's solution, and tissue culture media, and
2) a water soluble, metabolically inert substance which maintains density, osmolarity and pH in the presence of cells, optionally selected from the group 5-(N-2,3-dihydroxypropylacetamido) 2,4,5-tri-iodo-N,N'-bis(2,3-dihydroxypropyl) isophthalamide and Metrizamide ; mixed with a physiological cold storage solution, optionally selected from the group UW solution and Eurocollins, comprising
a) viscosity modifiers, optionally selected from colloids, optionally selected from the group hydroxyethyl starch and serum proteins; and polymers, optionally selected from the group polyethylene glycol, dextran and alginate; and
b) osmotically active impermeant agents for suppressing cold-induced cellular edema, optionally selected from sugars, optionally selected from the group glucose, mannitol, and sucrose; anions, optionally selected from the group phosphate, sulfate, glycerophosphate, citrate and gluconate; and compounds which supress cold induced cellular edema, optionally selected from lactobionate acid and raffinose;
overlayed with a physiological solution capable of maintaining viable cells, optionally selected from the group consisting of Hank's solution and tissue culture media.

2. The composition of claim 1 wherein the first and second solutions are slightly hypertonic to both cell types.

3. The composition of claim 1 wherein the islet and acinar cels are added to the first, or lower solution.

4. A method of isolating and purifying one first desired type of cells, being islet cells from a population containing a second type of cells, being acinar cells, having a different density from said first type after incubation in a first or lower solution, wherein said cells are optionally made more permeable by preferential digestion with enzymes, and said second type of cells optionally achieves a higher density than said first type of cells by being made more permeable, said method comprising:
A) adding said population of cells to said first, or lower solution, said first solution having a density greater than that of both cell types in their natural state, but less than that of the second cell type after incubation in said solution, optionally between 1.097 and 1.126 g/ml, and being slightly hypertonic to both cell types; said first solution comprising:
a water soluble, metabolically inert substance which maintains density, osmolarity and pH in the presence of cells, optionally selected from the group 5-(N-2,3-dihydroxypropylacetamido) 2,4,5-tri-iodo-N,N'-bis(2,3-dihydroxypropyl)-isophthalamide and Metrizamide ; mixed with a physiological cold storage solution, optionally selected from UW solution and Eurocollins, comprising viscosity modifiers, optionally selected from colloids, optionally selected from the group hydroxyethyl starch and serum proteins; and polymers, optionally selected from the group polyethylene glycol, dextran and alginate, to increase the viscosity of said first solution, and osmotically active impermeant agents, optionally selected from sugars, optionally selected from the group glucose, mannitol, and sucrose; anions, optionally selected from the group phosphate, sulfate, glycerophosphate, citrate and gluconate; and compounds which supress cold induced cellular edema, optionally selected from lactobionate acid and raffinose;
B) layering a second solution, having a density less than that of both cell types, and being slightly hypertonic to both cell types, selected from the group comprising:
1) a physiological solution capable of maintaining viable cells, optionally selected from Hank's solution, and tissue culture media, and
2) a water soluble, metabolically inert substance which maintains density, osmolarity and pH in the presence of cells, optionally selected from the group 5-(N-2,3-dihydroxypropylacetamido) 2,4,5-tri-iodo-N,N'-bis(2,3-dihydroxypropyl)-isophthalamide and Metrizamide ; mixed with a physiological cold storage solution, optionally selected from the group UW solution and Eurocollins, comprising viscosity modifiers, optionally selected from colloids, optionally selected from the group hydroxyethyl starch and serum proteins; and polymers, optionally selected from the group polyethylene glycol, dextran and alginate; and osmotically active impermeant agents, optionally selected from sugars, optionally selected from the group glucose, mannitol, and sucrose; anions, optionally selected from the group phosphate, sulfate, glycerophosphate, citrate and gluconate; and compounds which supress cold induced cellular edema, optionally selected from lactobionate acid and raffinose;
overlayed with a physiological solution capable of maintaining viable cells, optionally selected from the group consisting of Hank's solution and tissue culture media;
C) separating the two cell types by applying at least 1g force to said combination of solutions for sufficient time, to separate said two types of cells whereby said first, desired type of cells collect at or near the interface between said first solution and said second solution and said second type of cells accumulate at or near the bottom below said first solution; and
D) removing said first, desired type of cells.

5. The method of claim 4 wherein said force is between 1 g for 30 minutes and 180 g for 15 minutes at the interface.

6. The method of claim 4 wherein the acinar cells are made more permeable by directing collagenase enzymes to acinar cells via ductal injection of said enzymes into a pancreas.

## Patentansprüche

1. Eine zunächst als Zweiphasengradient vorhandene, bei der Isolierung und Reinigung von zwei Zelltypen unterschiedlicher Dichte nützliche wässrige Zusammensetzung, wobei ein erster Zelltyp, hier Azinuszellen, nach Inkubation in der ersten oder unteren Lösung eine höhere Dichte aufweist als ein zweiter Zelltyp, hier Inselzellen, wobei die Zusammensetzung umfaßt:
A)eine erste oder untere Lösung mit höchstens der Dichte des ersten Zelltyps, wahlweise zwischen 1,097 und 1,126 g/ml, wobei die erste Lösung umfaßt:
1) eine wasserlösliche, metabolisch inerte Substanz, die in Gegenwart von Zellen die Dichte, Osmolalität und den pH-Wert beibehält, beliebig ausgewählt aus der Gruppe 5-(N-2,3-Dihydroxypropylacetamido)-2,4,5-triiod-N,N'-bis(2,3-dihydroxypropyl)-isophthalamid und Metrizamid;
2)gemischt mit einer physiologischen kalten Lagerungslösung, wahlweise ausgewählt aus UW-Lösung oder Eurocollinen, umfassend
a) Viskositätsmodifikatoren, beliebig ausgewählt aus Kolloiden, beliebig ausgewählt aus der Gruppe Hydroxyethylstärke und Serumproteine; und Polymere, beliebig ausgewählt aus der Gruppe Polyethylenglykol, Dextran und Alginat; um die Viskosität der ersten Lösung zu erhöhen, und
b) osmotisch aktive Impermeantien, beliebig ausgewählt aus Zuckern, beliebig ausgewählt aus der Gruppe Glukose, Mannit und Saccharose, zur Unterdrückung eines durch Kälte ausgelösten Zellödems; beliebig ausgewählte Anionen aus der Gruppe Phosphat, Sulfat, Glyzerophosphat, Citrat und Glukonat; und Verbindungen, die ein durch Kälte ausgelöstes Zellödem unterdrücken, beliebig ausgewählt aus Lactobionsäure und Raffinose;
B)und eine zweite oder obere Lösung mit geringerer Dichte als der zweite Zelltyp, wahlweise weniger als 1,050 g/ml, wobei die zweite Lösung aus der Gruppe ausgewählt ist, die umfaßt:
1) eine physiologische Lösung, die zum Unterhalten lebensfähiger Zellen in der Lage ist und beliebig aus Hankscher Lösung und Gewebekulturmedien ausgewählt ist, und
2)eine wasserlösliche, metabolisch inerte Substanz, die in Gegenwart von Zellen die Dichte, Osmolalität und den pH-Wert beibehält, beliebig ausgewählt aus der Gruppe 5-(N-2,3-Dihydroxypropylacetamido)-2,4,5-triiod-N,N'-bis(2,3-dihydroxypropyl)-isophthalamid und Metrizamid; gemischt mit einer beliebig aus der Gruppe UW-Lösung und Eurocolline ausgewählten physiologischen kalten Lagerungslösung, umfassend
a) Viskositätsmodifikatoren, beliebig ausgewählt aus Kolloiden, beliebig ausgewählt aus der Gruppe Hydroxyethylstärke und Serumproteine; und Polymere, beliebig ausgewählt aus der Gruppe Polyethylenglykol, Dextran und Alginat; und
b) osmotisch aktive Impermeantien, beliebig ausgewählt aus Zuckern, beliebig ausgewählt aus der Gruppe Glukose, Mannit und Saccharose, zur Unterdrückung eines durch Kälte ausgelösten Zellödems; beliebig ausgewählte Anionen aus der Gruppe Phosphat, Sulfat, Glyzerophosphat, Citrat und Glukonat; und Verbindungen, die ein durch Kälte ausgelöstes Zellödem unterdrücken, beliebig ausgewählt aus Lactobionsäure und Raffinose;
überschichtet mit einer physiologischen Lösung, die zum Unterhalten lebensfähiger Zellen in der Lage ist und beliebig aus der Gruppe bestehend aus Hankscher Lösung und Gewebekulturmedien ausgewählt ist.

2. Zusammensetzung nach Anspruch 1, wobei die erste und die zweite Lösung gegenüber beiden Zelltypen etwas hypertonisch sind.

3. Zusammensetzung nach Anspruch 1, wobei die Insel- und Azinuszellen der ersten oder unteren Lösung zugefügt werden.

4. Verfahren zur Isolierung und Reinigung eines ersten gewünschten Zelltyps, hier Inselzellen, aus einer einen zweiten Zelltyp enthaltenden Population, hier Azinuszellen, die nach Inkubation in einer ersten oder unteren Lösung eine andere Dichte aufweisen als der erste Typ, wobei die Zellen durch bevorzugten Aufschluß mit Enzymen beliebig durchlässiger gemacht werden und der zweite Zelltyp dadurch, daß er durchlässiger gemacht wird, wahlweise eine höhere Dichte erreicht als der erste Zelltyp, wobei das Verfahren umfaßt:
A) Hinzufügen der Zellpopulation zu der ersten oder unteren Lösung, wobei die erste Lösung einer größere Dichte aufweist als beide Zelltypen in ihrem Naturzustand, aber eine geringere als der zweite Zelltyp nach Inkubation in der Lösung, wahlweise zwischen 1,097 und 1,126 g/ml und gegenüber beiden Zelltypen etwas hypertonisch; wobei die erste Lösung umfaßt:
eine wasserlösliche, metabolisch inerte Substanz, die in Gegenwart von Zellen die Dichte, Osmolalität und den pH-Wert beibehält, beliebig ausgewählt aus der Gruppe 5-(N-2,3-Dihydroxypropylacetamido)-2,4,5-triiod-N,N'-bis(2,3-dihydroxypropyl)-isophthalamid und Metrizamid; gemischt mit einer physiologischen kalten Lagerungslösung, wahlweise ausgewählt aus UW-Lösung oder Eurocollinen, mit Viskositätsmodifikatoren, beliebig ausgewählt aus Kolloiden aus der Gruppe Hydroxyethylstärke und Serumproteine; und Polymere, beliebig ausgewählt aus der Gruppe Polyethylenglykol, Dextran und Alginat, um die Viskosität der ersten Lösung zu erhöhen, und osmotisch aktive Impermeantien, beliebig ausgewählt aus Zuckern, beliebig ausgewählt aus der Gruppe Glukose, Mannit und Saccharose; beliebig ausgewählte Anionen aus der Gruppe Phosphat, Sulfat, Glyzerophosphat, Citrat und Glukonat; und Verbindungen, die ein durch Kälte ausgelöstes Zellödem unterdrücken, beliebig ausgewählt aus Lactobionsäure und Raffinose;
B)Darüberschichten einer zweiten Lösung, die eine geringere Dichte als beide Zelltypen aufweist und gegenüber beiden Zelltypen etwas hypertonisch ist und aus der Gruppe ausgewählt ist, die umfaßt:
1) eine physiologische Lösung, die zum Unterhalten lebensfähiger Zellen in der Lage ist und beliebig aus Hankscher Lösung und Gewebekulturmedien ausgewählt ist, und
2)eine wasserlösliche, metabolisch inerte Substanz, die in Gegenwart von Zellen die Dichte, Osmolalität und den pH-Wert beibehält, beliebig ausgewählt aus der Gruppe 5-(N-2,3-Dihydroxypropylacetamido)-2,4,5-triiod-N,N'-bis(2,3-dihydroxypropyl)-isophthalamid und Metrizamid; gemischt mit einer beliebig aus der Gruppe UW-Lösung und Eurocolline ausgewählten physiologischen kalten Lagerungslösung, mit Viskositätsmodifikatoren, beliebig ausgewählt aus Kolloiden aus der Gruppe Hydroxyethylstärke und Serumproteine; und Polymere, beliebig ausgewählt aus der Gruppe Polyethylenglykol, Dextran und Alginat, und osmotisch aktive Impermeantien, beliebig ausgewählt aus Zuckern, beliebig ausgewählt aus der Gruppe Glukose, Mannit und Saccharose; beliebig ausgewählte Anionen aus der Gruppe Phosphat, Sulfat, Glyzerophosphat, Citrat und Glukonat; und Verbindungen, die ein durch Kälte ausgelöstes Zellödem unterdrücken, beliebig ausgewählt aus Lactobionsäure und Raffinose;
überschichtet mit einer physiologischen Lösung, die zum Unterhalten lebensfähiger Zellen in der Lage ist und beliebig aus der Gruppe bestehend aus Hankscher Lösung und Gewebekulturmedien ausgewählt ist.
C)Trennen der zwei Zelltypen durch ausreichend langes Anlegen einer Kraft von mindestens 1 g auf die Kombination der Lösungen zur Trennung der zwei Zelltypen, wobei sich der erste, gewünschte Zelltyp an oder nahe an der Grenzfläche zwischen der ersten Lösung und der zweiten Lösung sammelt und sich der zweite Zelltyp am oder nahe am Boden unter der ersten Lösung anhäuft; und
D)Entnehmen des ersten, gewünschten Zelltyps.

5. Verfahren nach Anspruch 4, wobei die Kraft zwischen 1 g für 30 Minuten und 180 g für 15 Minuten an der Grenzfläche beträgt.

6. Verfahren nach Anspruch 4, wobei die Azinuszellen dadurch durchlässiger gemacht werden, daß Kollagenase-Enzyme mittels Injektion der Enzyme in den Gang einer Bauchspeicheldrüse auf Azinuszellen gerichtet werden.

## Revendications

1. Composition aqueuse, se présentant au départ sous forme d'un gradient à deux phases, utile dans l'isolement et la purification de deux types cellulaires de densités différentes, dans laquelle un premier type cellulaire, consistant en cellules acineuses, présente une densité plus élevée qu'un second type cellulaire, consistant en cellules insulaires, après incubation dans la première solution ou solution inférieure, ladite composition comprenant :
A) une première solution, ou solution inférieure ayant une densité non supérieure à celle du premier type cellulaire, éventuellement comprise entre 1,097 et 1,126 g/ml, ladite première solution comprenant :
1) une substance métaboliquement inerte, soluble dans l'eau, qui assure aux cellules un milieu de densité, d'osmolarité et de *p*H constants, choisie le cas échéant dans le groupe de 5-(N-2, 3-dihydroxypropylacétamido) 2, 4, 5-tri-iodo-N, N'-bis(2, 3-dihydroxypropyl)isophtalamide et de Métrizamide;
2) en mélange avec une solution de conservation physiologique à froid, choisie le cas échéant parmi la solution UW et les Eurocollins, comprenant
a) des modificateurs de la viscosité, éventuellement choisis parmi les colloïdes, ceux-ci étant choisis le cas échéant dans le groupe d'hydroxyéthyl amidon et de protéines sériques; et des polymères, choisis éventuellement dans le groupe de polyéthylène-glycol, de dextrane et d'alginate; en vue d'accroître la viscosité de ladite première solution; et b) des agents doués d'activité osmotique non pénétrants ou à faible pouvoir de pénétration, destinés à supprimer le gonflement cellulaire sous l'effet du froid, choisis éventuellement parmi les sucres, ceux-ci étant choisis le cas échéant parmi le glucose, le mannitol, et le saccharose; des anions, choisis éventuellement parmi le groupe de phosphate, de sulfate, de glycérophosphate, de citrate et de gluconate, et des composés qui suppriment le gonflement cellulaire sous l'effet du froid, éventuellement choisis parmi le lactobionate et le raffinose;
B) et une seconde solution, ou solution supérieure ayant une densité inférieure à celle du second type cellulaire, éventuellement inférieure à 1,050 g/ml, ladite seconde solution étant choisie dans le groupe comprenant :
1) une solution physiologique capable de maintenir les cellules dans un état viable, choisie le cas échéant parmi la solution de Hank, et les milieux de culture tissulaire, et
2) une substance métaboliquement inerte, soluble dans l'eau, qui assure aux cellules un milieu de densité, d'osmolarité et de *p*H constants, choisie le cas échéant dans le groupe de 5-(N-2, 3-dihydroxypropylacétamido) 2, 4, 5-tri-iodo-N, N'-bis(2, 3-dihydroxypropyl)isophtalamide et de Métrizamide en mélange avec une solution de conservation physiologique à froid, choisie le cas échéant parmi le groupe de solutions UW et des Eurocollins, comprenant
a) des modificateurs de la viscosité, éventuellement choisis parmi les colloïdes, ceux-ci étant choisis le cas échéant dans le groupe d'hydroxyéthyl amidon et de protéines sériques; et des polymères choisis éventuellement dans le groupe de polyéthylène-glycol, de dextrane et d'alginate; et
b) des agents doués d'activité osmotique non pénétrants ou à faible pouvoir de pénétration, destinés à supprimer le gonflement cellulaire sous l'effet du froid, choisis éventuellement parmi les sucres, ceux-ci étant choisis le cas échéant parmi le groupe de glucose, de mannitol, et de saccharose; des anions, choisis éventuellement parmi le groupe de phosphate, de sulfate, de glycérophosphate, de citrate et de gluconate, et des composés qui suppriment le gonflement cellulaire sous l'effet du froid, éventuellement choisis parmi le lactobionate et le raffinose;
solution sur laquelle se trouve superposée une solution physiologique capable de maintenir les cellules dans un état viable, choisie le cas échéant dans le groupe constitué de solution de Hank et de milieux de culture tissulaire.

2. Composition selon la revendication 1, dans laquelle les premières et secondes solutions sont légèrement hypertoniques vis-à-vis des deux types cellulaires

3. Composition selon la revendication 1, dans laquelle les cellules insulaires et acineuses sont ajoutées à la première solution, ou solution inférieure.

4. Procédé d'isolement et de purification d'un premier type cellulaire recherché, consistant en cellules insulaires, à partir d'une population contenant un second type cellulaire, consistant en cellules acineuses, ayant une densité différente de celle du premier type cellulaire après incubation dans une première solution ou solution inférieure, procédé dans lequel lesdites cellules sont éventuellement rendues plus perméables par digestion sélective au moyen d'enzymes, et dans lequel ledit second type cellulaire atteint éventuellement une densité supérieure à celle dudit premier type cellulaire en aquérant une plus grande perméabilité, ledit procédé comprenant :
A) l'adjonction de ladite population de cellules à ladite première solution ou solution inférieure, ladite première solution ayant une densité supérieure à celle des deux types cellulaires à l'état natif, mais inférieure à celle du second type cellulaire après incubation dans ladite seconde solution, densité éventuellement comprise entre 1,097 et 1,126 g/ml, et étant légèrement hypertonique vis-à-vis des deux types cellulaires; ladite première solution comprenant :
une substance métaboliquement inerte, soluble dans l'eau, qui assure aux cellules un milieu de densité, d'osmolarité et de *p*H constants, choisie le cas échéant dans le groupe de 5-(N-2, 3-dihydroxypropylacétamido) 2, 4, 5-tri-iodo-N, N'-bis(2, 3-dihydroxypropyl)isophtalamide et de Métrizamide; en mélange avec une solution de conservation physiologique à froid, choisie le cas échéant parmi la solution UW et les Eurocollins, comprenant des modificateurs de la viscosité, choisis éventuellement parmi les colloïdes, ceux-ci étant choisis le cas échéant dans le groupe d'hydroxyéthyl amidon et de protéines sériques; et des polymères choisis éventuellement dans le groupe de polyéthylène-glycol, de dextrane et d'alginate, en vue d'accroître la viscosité de ladite première solution; et des agents doués d'activité osmotique non pénétrants ou à faible pouvoir de pénétration choisis éventuellement parmi les sucres, ceux-ci étant choisis le cas échéant dans le groupe de glucose, de mannitol, et de saccharose; des anions, éventuellement choisis parmi le groupe de phosphate, de sulfate, de glycérophosphate, de citrate et de gluconate; et des composés qui suppriment le gonflement cellulaire sous l'effet du froid, éventuellement choisis parmi le lactobionate et le raffinose;
B) la superposition d'une seconde solution, ayant une densité inférieure à celle des deux types cellulaires tout en étant légèrement hypertonique vis-à-vis des deux types cellulaires, choisie dans le groupe comprenant :
1) une solution physiologique capable de maintenir les cellules dans un état viable, choisie le cas échéant parmi la solution de Hank, et les milieux de culture tissulaire, et
2) une substance métaboliquement inerte, soluble dans l'eau, qui assure aux cellules un milieu de densité, d'osmolarité et de *p*H constants, choisie le cas échéant dans le groupe de 5-(N-2, 3-dihydroxypropylacétamido) 2, 4, 5-tri-iodo-N, N'-bis(2, 3-dihydroxypropyl)isophtalamide et de Métrazamide; en mélange avec une solution de conservation physiologique à froid, choisie le cas échéant parmi le groupe de solutions UW et des Eurocollins, comprenant des modificateurs de la viscosité, choisis éventuellement parmi les colloïdes, ceux-ci étant choisis le cas échéant dans le groupe d'hydroxyéthyl amidon et de protéines sériques; et des polymères choisis éventuellement dans le groupe de polyéthylène-glycol, de dextrane et d'alginate; et des agents doués d'activité osmotique non pénétrants ou à faible pouvoir de pénétration, éventuellement choisis parmi les sucres, ceux-ci étant choisis le cas échéant dans le groupe de glucose, de mannitol, et de saccharose; des anions, choisis éventuellement dans le groupe de phosphate, de sulfate, de glycérophosphate, de citrate et de gluconate; et des composés qui suppriment le gonflement cellulaire sous l'effet du froid, éventuellement choisis parmi le lactobionate et le raffinose;
solution sur laquelle se trouve superposée une solution physiologique capable de maintenir les cellules dans un état viable, choisie le cas échéant parmi la solution de Hank et les milieux de culture tissulaire,
C) la séparation des deux types cellulaires en appliquant une force au moins égale à 1 g à ladite combinaison de solutions pour une durée suffisamment longue, en vue de séparer lesdits deux types cellulaires de manière à ce que le premier type cellulaire recherché s'accumule au niveau ou à proximité de l'interphase entre ladite première et ladite seconde solution et à ce que le second type cellulaire s'accumule au fond ou à proximité du fond en dessous de ladite première solution; et
D) le retrait dudit premier type cellulaire recherché.

5. Procédé selon la revendication 4, dans lequel ladite force est comprise entre lg pour une durée de 30 minutes et 180 g pour une durée de 15 minutes au niveau de l'interphase.

6. Procédé selon la revendication 4, dans lequel les cellules acineuses sont rendues plus perméables en délivrant des enzymes de type collagénase aux cellules acineuses par injection intratubulaire desdites enzymes dans le pancréas.
